# EUROPEAN PATENT SPECIFICATION

(11) **EP 1 294 663 B1**
(45) Date of publication and mention of the grant of the patent: **06.05.2004**
(21) Application number: 00941373.3
(22) Date of filing: 12.06.2000
(51) Int. Cl.: C07C 17/20, C07C 25/13

(54) **FLUORINATED BENZENE MANUFACTURING PROCESS**
VERFAHREN ZUR HERSTELLUNG VON FLUORIERTEN BENZOLEN
PROCEDE DE PRODUCTION DE BENZENE FLUORE

(43) Date of publication of application: 26.03.2003
(73) Proprietor: University of Florida Research Foundation, Gainesville, FL 32611-5500 (US)
(72) Inventor: SUBRAMANIAN, Munirpallam, A., Kennett Square, PA 19348 (US)
(74) Representative: Ferreccio, Rinaldo
(86) International application number: PCT/US2000/016129
(87) International publication number: WO 2001/096267

(56) References cited:
- GB-A- 1 039 247
- US-A- 4 394 527
- US-A- 6 087 543

## Description

This invention relates to a process for the manufacture of fluorobenzene by contacting chlorobenzene with argentous fluoride.

### BACKGROUND

Fluorobenzene, an agricultural chemicals intermediate, is typically produced by the reaction of aniline and sodium nitrite in the presence of hydrogen fluoride. A diazonium salt intermediate is formed during this process which because of its instability adds to the cost of manufacture. U.S. Patent No. 4,394,527 discloses a process for monofluorinating a benzene nucleus comprising reacting a benzene compound in the liquid phase with argentic fluoride which is reduced to argentous fluoride during the reaction.

There is still a need for an efficient commercial process for preparing fluorobenzene using less expensive materials.

### SUMMARY OF THE INVENTION

A process is provided for producing fluorinated benzene. The process comprises (a) contacting chlorobenzene starting material with a metal fluoride composition of the formula (AgF)(MF₂)ₓ where M is selected from the group consisting of Mn, Fe, Co, Ni, Cu, Zn and mixtures thereof and wherein x is a number between 0 and 1, at a temperature above 175°C sufficient to remove the chlorine substituent from the starting material and to transfer F from the metal fluoride composition to the starting material, thereby producing a reduced metal fluoride composition comprising a silver component of the formula AgF_{1-y} where y is a number from 0.01 to 1; (b) oxidizing the reduced metal fluoride composition from (a) in the presence of HF to regenerate the metal fluoride mixture composition of the formula (AgF)(MF₂)ₓ; and (c) recycling regenerated metal fluoride composition of (b) to (a).

### DETAILED DESCRIPTION

An important aspect of this invention involves the reaction of a metal fluoride composition of the formula (AgF)(MF₂)ₓ, where M and x are as defined above, with chlorobenzene to produce fluorobenzene. In an embodiment of this invention chlorobenzene is passed over the regenerable reagent, argentous fluoride (AgF), at reaction conditions until the conversion rate to fluorobenzene is reduced to an economically insufficient level. The contacting of chlorobenzene (C₆H₅Cl) with argentous fluoride is done in the vapor phase at a temperature from 175°C to 220°C, preferably from 200°C to 220°C. As the reaction temperature is raised above 220°C, the fluorobenzene (C₆H₅F) is further fluorinated and difluorobenzene (C₆H₄F₂), trifluorobenzene (C₆H₃F₃) and tetrafluorobenzene (C₆H₂F₄) are produced.

In a second embodiment, chlorobenzene is passed over the regenerable reagent, (AgF)(MF₂)ₓ, at reaction conditions until the conversion rate to fluorobenzene is reduced to an economically insufficient level. With this second reagent the contacting with chlorobenzene is also done in the vapor phase, but at a temperature of from 250°C to 450°C, preferably from 275°C to 325°C. As the reaction temperature is raised above 300°C, the fluorobenzene (C₆H₅F) is further fluorinated and difluorobenzene (C₆H₄F₂), trifluorobenzene (C₆H₃F₃) and tetrafluorobenzene (C₆H₂F₄) are produced.

The (AgF)(MF₂)ₓ functions as a regenerable fluorinating reagent (i.e., the reduced metal fluoride composition comprising a reduced form of silver such as metallic silver can be oxidized back to (AgF)(MF₂)ₓ). The argentous fluoride (AgF) can be used by itself or as part of a mixture. The metal fluoride mixtures of this invention, (AgF)(MF₂)ₓ where M is selected from the group consisting of Mn, Fe, Co, Ni, Cu, Zn and/or mixtures thereof and wherein x is a number between 0 and 1 can be prepared by conventional engineering mixing techniques using the metal fluoride powder(s). Mixed metal compounds such as AgMnF₃, AgFeF₃, AgCoF₃, AgNiF₃, AgCuF₃ and AgZnF₃ can be prepared by heating a 1:1 molar mixture of AgF and MF₂, where M is as defined above, to between from 400°C to 450°C for at least one hour in an inert atmosphere (e.g., nitrogen or argon). The powders may be made into granules or pellets.

The contact time is typically from 1 to 120 seconds (e.g., from 5 to 60 seconds).

The reaction can also be done in the presence of inert gases which are stable under the reaction conditions such as nitrogen and argon.

Unreacted chlorobenzene can be recycled to the reactor for the production of additional fluorobenzene. The fluorobenzene may be recovered from the reaction product and any unreacted benzene by conventional procedures such as distillation.

Argentous fluoride can be regenerated from the fluoride-depleted reagent either by reacting with oxygen and HF at a temperature between 250°C to 500°C or by converting the fluoride-depleted reagent to a stable salt (e.g, AgNO₃) and reacting said salt with HF. The oxygen may be diluted with inert gases such nitrogen and argon.

The reaction zone and its associated feed lines, effluent lines and associated units should be constructed of materials resistant to hydrogen fluoride. Typical materials of construction, well-known to the fluorination art, include stainless steels, in particular of the austenitic type, the well-known high nickel alloys, such as Monel® nickel-copper alloys, Hastelloy® nickel-based alloys and, Inconel® nickel-chromium alloys, and copper-clad steel. Silicon carbide is also suitable for reactor fabrication.

Without further elaboration, it is believed that one skilled in the art can, using the description herein, utilize the present invention to its fullest extent. The following embodiments are to be construed as illustrative, and not as constraining the remainder of the disclosure in any way whatsoever.

### EXAMPLES

### EXAMPLE 1

### Preparation of Fluorobenzene

An Inconel® nickel alloy tube reactor was packed with argentous fluoride (AgF, 5 g). The catalyst was heated to reaction temperature under a nitrogen flow. The nitrogen flow was adjusted to 30 cc/min. passed through chlorobenzene over the catalyst. Reaction products were analyzed using a Hewlett Packard 6890 Gas Chromatograph/5973Mass Spectrometer. All analyses are reported in area% and are shown in Table 1.

**TABLE 1**

| Run No. | T (°C) | %C₆H₅Cl | %C₆H₅F | %C₆H₄F₂ | %C₆H₃F₃ | %C₆H₂F₄ |
|---|---|---|---|---|---|---|
| 1 | 200 | 75.1 | 20.4 | - | - | - |
| 2 | 210 | 77.3 | 22.7 | - | - | - |
| 3 | 220 | 68.7 | 26.3 | 5 | - | - |
| 4 | 230 | 60.5 | 33.5 | 6 | - | - |
| 5 | 240 | 42.8 | 47 | 10.2 | - | - |
| 6 | 250 | 28.5 | 56.7 | 9.6 | - | - |
| 7 | 260 | 9.4 | 70.1 | 20.5 | - | - |
| 8 | 270 | 4.2 | 67 | 28.7 | - | - |
| 9 | 280 | <0.1 | 58.8 | 36.2 | 5 | - |
| 10 | 290 | <0.1 | 52.2 | 41 | 6.8 | <0.1 |
| 11 | 300 | <0.1 | 45.5 | 46.3 | 8.2 | <0.1 |
| 12 | 310 | <0.1 | 37.4 | 49.1 | 13.6 | <0.1 |

### EXAMPLE 2

An Inconel® nickel alloy tube reactor was packed with AgCuF₃ (5 g). The catalyst was heated to reaction temperature under a nitrogen flow. The nitrogen flow was adjusted to 30 cc/min. and passed through chlorobenzene over the catalyst. Reaction products were analyzed using a Hewlett Packard 6890 Gas Chromatograph/5973Mass Spectrometer. All analyses are reported in area% and are shown in Table 2.

**TABLE 2**

| Run No. | T (°C) | %C₆H₅Cl | %C₆H₅F | %C₆H₄F₂ | %C₆H₄ClF | %C₆H₂F₄ |
|---|---|---|---|---|---|---|
| 1 | 250 | 99.9 | <0.1 | - | - | - |
| 2 | 300 | 84.2 | 15.8 | - | <0.1 | - |
| 3 | 350 | 44.8 | 34.8 | 9.9 | 1.7 | - |
| 4 | 400 | 23.2 | 39 | 37.8 | <0.1 | - |
| 5 | 450 | 21.2 | 29 | 44.9 | - | 5^{a} |

| | | | | | | |
|---|---|---|---|---|---|---|
| ^{a}C₆H₃F₃ (<0.1%) was also detected | | | | | | |

## Claims

1. A process for producing fluorinated benzene, comprising:
(a) contacting chlorobenzene starting material with a metal fluoride composition of the formula (AgF)(MF₂)ₓ where M is selected from the group consisting of Mn, Fe, Co, Ni, Cu, Zn and mixtures thereof and wherein x is a number between 0 and 1, at a temperature above 175°C sufficient to remove the chlorine substituent from the starting material and to transfer F from the metal fluoride composition to the starting material, thereby producing a reduced metal fluoride composition comprising a silver component of the formula AgF_{1-y} where y is a number from 0.01 to 1;
(b) oxidizing the reduced metal fluoride composition from (a) in the presence of HF to regenerate the metal fluoride mixture composition of the formula (AgF)(MF₂)ₓ; and
(c) recycling regenerated metal fluoride composition of (b) to (a).

2. The process of Claim 1 wherein fluorobenzene is produced by contacting chlorobenzene with argentous fluoride.

## Patentansprüche

1. Ein Verfahren zum Herstellen von fluoriertem Benzol, welches umfasst:
(a) in Kontakt bringen einer Chlorbenzol-Ausgangssubstanz mit einer Metallfluoridzusammensetzung der Formel (AgF) (MF₂)ₓ, wobei M aus der Gruppe Mn, Fe, Co, Ni, Cu, Zn und deren Mischungen ausgewählt ist und wobei x eine Zahl zwischen 0 und 1 ist, bei einer Temperatur oberhalb 175°C, die ausreicht, um den Chlor-Substituenten von der Ausgangssubstanz zu lösen und F von der Metallfluoridzusammensetzung zur Ausgangssubstanz zu übertragen und dadurch eine reduzierte Metallfluoridzusammensetzung herzustellen, die eine Silberverbindung der Formel AgF_{1-y} umfasst, wobei y eine Zahl von 0,01 bis 1 ist;
(b) Oxidieren der reduzierten Metallfluoridzusammensetzung aus Schritt (a) in Gegenwart von HF, um die Metallfluoridzusammensetzung der Formel (AgF)(MF₂)ₓ zu regenerieren; und
(c) Rückführen der in Schritt (b) regenerierten Metallfluoridzusammensetzung in den Schritt (a).

2. Das Verfahren nach Anspruch 1, wobei Fluorobenzol durch in Kontakt bringen von Chlorbenzol mit Silberfluorid hergestellt wird.

## Revendications

1. Procédé pour produire du fluorobenzene, comprenant les étapes de :
(a) Faire réagir du chlorobenzene comme produit de départ avec un complexe de fluorure métallique de formule (AgF)(MF₂)ₓ où M est sélectionné à partir du groupe comprenant le Mn,le Fe, le Co, le Ni, le Cu et le Zn et leurs combinaisons, et où x est compris entre 0 et 1, à une température au dessus de 175°C suffisante pour remplacer le substituant chloré du produit de départ et pour transférer du F du complexe de fluorure métallique vers le produit de départ, produisant ainsi un complexe de fluorure métallique réduit comprenant un composé d'argent de formule AgF_{1-y} où y est un nombre compris entre 0,01 et 1.
(b) Oxyder le complexe de fluorure métallique réduit tel que défini en (a) en présence de HF pour régénérer le complexe de mélange de fluorure métallique et de métal de formule (AgF)(MF₂)ₓ ; et
(c) Recycler le complexe de fluorure métallique obtenu en (b) dans (a)

2. Procédé selon la revendication 1 **caractérisé en ce qu'**on prépare le fluorobenzene par réaction du chlorobenzene avec du fluorure d'argent.
